# EUROPÄISCHE PATENTSCHRIFT

(11) **EP 1 233 747 B1**
(45) Veröffentlichungstag und Bekanntmachung des Hinweises auf die Patenterteilung: **15.09.2004**
(21) Anmeldenummer: 00979620.2
(22) Anmeldetag: 23.11.2000
(51) Int. Cl.: A61K 7/48, A61K 31/35

(54) **VERWENDUNG VON FLAVONEN UND/ODER ISOFLAVONEN AUS PFLANZENEXTRAKTEN**
USE OF FLAVONES AND/OR ISOFLAVONES FROM PLANT EXTRACTS
UTILISATION DE FLAVONES ET/OU D'ISOFLAVONES TIREES D'EXTRAITS DE PLANTES

(30) Priorität: 02.12.1999 EP 99440342
(43) Veröffentlichungstag der Anmeldung: 28.08.2002
(73) Patentinhaber: Cognis France S.A., 31360 Saint-Martory (FR)
(72) Erfinder: PAULY, Gilles, F-54000 Nancy (FR); MOSER, Philippe, F-54270 Essey-les-Nancy (FR)
(74) Vertreter: Fabry, Bernd, Dr.
(86) Internationale Anmeldenummer: PCT/EP2000/011661
(87) Internationale Veröffentlichungsnummer: WO 2001/039738

(56) Entgegenhaltungen:
- EP-A- 0 681 827
- EP-A- 0 781 544
- WO-A-00/32062
- WO-A-00/33824
- WO-A-98/13020
- WO-A-99/25316
- FR-A- 2 719 473
- US-A- 5 665 367
- US-A- 5 952 373
- US-A- 5 972 993
- DATABASE CAPLUS [Online] CHEMICAL ABSTRACTS SERVICE, COLUMBUS, OHIO, US; retrieved from STN Database accession no. 1995:997755 XP002167393 & CN 1 104 246 A (PEOP. REP. CHINA) 28. Juni 1995 (1995-06-28)
- DATABASE CAPLUS [Online] CHEMICAL ABSTRACTS SERVICE, COLUMBUS, OHIO, US; retrieved from STN Database accession no. 1996:128449 XP002167394 & JP 07 324025 A (SANKODO KK) 12. Dezember 1995 (1995-12-12)
- PATENT ABSTRACTS OF JAPAN vol. 1996, no. 09 (C & JP 08 133940 A (POLA CHEM IND CO)
- DATABASE WPI Week 199816 Derwent Publications Ltd., London, GB; AN 1998-174834 XP002137767 & JP 10 036279 A (ICHIMARU PHARCOS INC) in der Anmeldung erwähnt
- DATABASE WPI Week 199711 Derwent Publications Ltd., London, GB; AN 1997-115211 XP002137768 & JP 09 002964 A (SANKI SHOJI KK)
- DATABASE WPI Week 199919 Derwent Publications Ltd., London, GB; AN 1999-226071 XP002137769 & JP 11 060458 A (EIKODO KK)
- DATABASE WPI Week 199913 Derwent Publications Ltd., London, GB; AN 1999-148433 XP002137770 & JP 11 012122 A (POLA CHEM IND INC)
- PATENT ABSTRACTS OF JAPAN vol. 015, no. 332 (C-0861) & JP 03 127714 A (KOBAYASHI KOSE CO)

## Beschreibung

### Gebiet der Erfindung

Die Erfindung befindet sich auf dem Gebiet der kosmetischen Haut- und Haarbehandlung und betrifft zum einen die Verwendung spezieller Pflanzenextrakten mit einem Gehalt an Flavonen und/oder Isoflavonen sowie spezielle Mischungen dieser Flavone und/oder Isoflavone und deren Verwendung in der Haut- und/oder Haarbehandlung.

### Stand der Technik

Seit längerer Zeit ist bekannt, daß Sexual- bzw. Steroidhormone vom Androgen- bzw. Oestrogentyp einen Einfluß auf das Wachstum von Keratinocyten haben und die epitheliale Zelldifferenzierung in der Epidermis beeinflussen. Unmittelbar in Zusammenhang steht damit die Biosynthese und die Umwandlung von Kollagen in den obersten Hautschichten. Zu den gesicherten Erkenntnissen zählt ferner, daß der weibliche Organismus in der Prae-Menopause eine signifikante Abnahme des Progesteronspiegels erleidet, dem in der Menopause dann eine Abnahme des Oestrogenspiegels folgt. Dies führt dazu, daß infolge mangelnder Verfügbarkeit an entsprechenden Sexualhormonen die Stimulation der Keratinocyten erheblich vermindert wird, was dazu führt, daß beispielsweise weniger Kollagen gebildet wird. Im Endergebnis verliert die Haut ihre Spannkraft, bildet Falten und wird subjektiv als "alt" empfunden. Zwar kann das Absinken des Hormonspiegels durch orale Verabreichung beispielsweise von Oestrogenen ausgeglichen werden, die Praxis zeigt jedoch, daß nur ein kleiner Teil der Verbraucherinnen hierzu bereit ist.

Demzufolge hat die Aufgabe der vorliegenden Erfindung darin bestanden, neue kosmetische Wirkstoffe zur Verfügung zu stellen, welche bei topischer Anwendung die Keratinocyten stimulieren und damit einen Ausgleich für den abgesunkenen Sexualhormonspiegel darstellen. Insbesondere sollten die neuen Wirkstoffe in der Anwendung dafür Sorge tragen, dass gerade ältere Haut jünger erscheint, d.h. dass Falten geglättet und die Spannkraft erhöht wird. Gleichzeitig sollten die Wirkstoffe als Radikalfänger wirken, also den Einfluss von Umweltgiften und den negativen Einfluss anderer Umwelteinflüsse wie z.B. der Sonneneinstrahlung auf die Haut und auf Haare mildern, und wenigstens überwiegend pflanzlicher Herkunft sein.

### Beschreibung der Erfindung

Gegenstand der Erfindung ist die Verwendung von Flavonen und/oder Isoflavonen aus Extrakten der Pflanzen, die ausgewählt sind aus der Gruppe die gebildet wird von Prunus, Adansonia und Citrus in kosmetischen Mitteln für Haut- und Haarbehandlung.

Überraschenderweise wurde gefunden, daß Flavone und/oder Isoflavone, vorzugsweise Gemische von Genisteinen und Naringeninen aus Prunus Extrakten, Adansonia-Extrakten oder aus Citrus-Extrakten auf die Tätigkeit von Keratinocyten bzw. Fibroblasten sowie die Synthese von Kollagen gerade in älterer Haut einen stimulierenden Einfluß hat. Gleichzeitig wirken sie als Radikalfänger bzw. Antioxidantien, was die Extrakte zur Herstellung von Sonnenschutzmitteln geeignet macht. In Summe führt die Verwendung von Flavonen und/oder Isoflavonen zum einen dazu, das die Hautelastizität verstärkt wird und die Haut insgesamt jünger erscheint, und zum anderen dazu, dass kosmetische Mittel, die Flavone und/oder Isoflavone aus den genannten Pflanzen enthalten, als Sonnenschutzmittel sowohl für die Haut als auch für die Haare wirksam sind.

### Adansonia Extrakte:

Pflanzen der Gattung Adansonia stammen vermutlich aus Madagaskar, wo mehrere einheimische Arten beschrieben wurden. Andere Arten der genannten Gattung wurden in Ostafrika und in Australien gefunden.
Die unterschiedlichen Bestandteile einer Art der Gattung Adansonia, der Art Adansonia digitata oder Affenbrotbaum werden nach wie vor in Afrika verwendet und genutzt, entweder ökonomisch (Rinde zur Herstellung von Seilen und Papier, Fruchtfleisch als Koagulationsmittel für Kautschuk und Wurzeln als roten Farbstoff) oder als Nahrungsmittel (insbesondere die Samen und jungen Blätter) oder sogar zur medizinischen Verwendung (die Rinde weist astringierende, diaphoretische und sogar fiebersenkende Eigenschaften auf; das Fruchtfleisch und die Samen weisen Eigenschaften gegen Dysenterie sowie entzündungshemmende Eigenschaften auf; die Blätter werden gegen das Schwitzen, gegen Nierenund Hamblasenbeschwerden und als Antiasthmatikum und Aufweichmittel verwendet).
Affenbrotbaumblätter enthalten neben flavonoidartigen Verbindungen auch Schleimstoffe Mineralsalze, Proteine, Catechingerbstoffe und Vitaminverbindungen (Riboflavin, Thiamin, Vitamin C, Niacin); (YAZZIE D et al., Joumal of Food Composition and Analysis, 1994, 7;3, 198-193 - GAIWE R et al., International Journal of Crude Drug Research, 1989, 27, 2, 101-104).
Die Extrakte können in an sich bekannter Weise, beispielsweise in Form wäßriger, alkoholischer oder wäßrig/alkoholischer Auszüge gewonnen werden.

### Citrus-Extrakte:

Die Citrus-Arten zählen zur botanischen Gattung der Rutaceen. Diese werden in zahlreichen Züchtungsformen kultiviert. Die wichtigsten Citrus-Arten sind: C. aurantium (Sauer- od. Bitterorange, Pomeranze, Bigarade, Bergamotte), C. aurantifolia (Limette), C. grandis bzw. paradisi (Pampelmuse, Shaddock bzw. Grapefruit), C. reticulata (Mandarine, Clementine, Tangerine), C. limonia (Limone od. Echte Zitrone), C. medica (Zedrat-Zitrone, liefert Zitronat) u. C. sinensis (Apfelsine, Orange, Süßorange). Daneben gibt es zahlreiche, z. T. mit Phantasienamen belegte Kreuzungen, z. B. die Citrange (Zitrange; Kreuzung von C. sinensis mit dem Pollen von Poncirus trifoliata, Synonym C. trifoliata) sowie Zitrangequat, Limequat, Orangequat (Kreuzungen mit Kumquat), Tangelo (aus Mandarine u. Grapefruit) und anderen. Verwandt mit den Citrus-Arten sind die Fortunella-Arten: Zwerg-C. mit süßen Schalen und saurem Fruchtinneren (Kumquat). Der Citronenbaum (Citrus limon), wird in Mittelmeerländem, Kalifornien, West- und Ostindien in verschiedenen Rassen kultiviert. Der Citronenbaum blüht das ganze Jahr; er trägt oft gleichzeitig Blüten und Früchte und gibt im Jahr 3-4 Ernten.

Vielfältige Verwendung der Citrus-Arten finden zum Beispiel das Fruchtfleisch bzw. der durch Auspressen gewonnene Saft (auch wegen des Gehalts an Ascorbinsäure) als Nahrungsmittel bzw. Getränk sowie Schalen, Blüten u. a. Pflanzenteile zur Gewinnung von Citrusölen.

Je 100 g essbare Citronenanteile enthalten durchschnittlich 90 g Wasser, 0,7 g Eiweiß, 0,6 g Fett, 7,1 g Kohlenhydrate, 0,9 g Rohfaser, 0,5 g Mineralstoffe, 53 mg Vitamin C; Zitronensaft enthält 4,7-7,9% Citronensäure u. 0,12-1,57% Invertzucker. Die Schale enthält etherische Öle, Bitterstoffe (Limonin), Hesperidin und andere Flavone, Isoflavone und Flavon-glykoside, Gerbstoff.

Die Herstellung der erfindungsgemäß einzusetzenden Citrus-Extrakte erfolgt durch übliche Methoden der Extraktion von Pflanzen bzw. Pflanzenteilen. Bevorzugt werden die Früchte und Fruchtschalen der Citrus-Arten extrahiert.

Im Sinne der vorliegenden Erfindung werden unter Flavonen und Isoflavonen solche verstanden, die sich aus Prunus, Adansonia und/oder Citrus extrahieren lassen. Sie umfassen Flavone und Isoflavone im engeren Sinne als auch ihre Derivate wie beispielsweise Flavane, Flavan-3-ole (Catechine), Flavan-3,4-diole (Leukoanthocyanidine), Flavonole und Flavanone. Typische Vertreter, die in den Extrakten aus Prunus, Adansonia und Citrus zu finden sind, stellen folgende Stoffe dar: Dihydromorin, Genistein (I), Genistin (Genistein-7-O-glucosid, II) Naringenin (III), Salipurposid (Naringenin-glycosid), Prunin (Naringenin-7-O-glucosid), Sakuranetin (Naringenin-7-methylether), Dihydrowogonin, Dihydrowogonin-7-O-glucosid, Chrysin, Chrysin-7-O-glucosid, Rutin, Quercetin, Galangin, Catechin und Epicatechin. 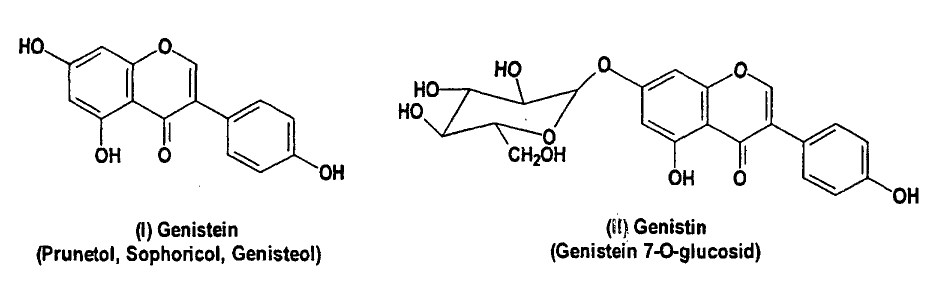 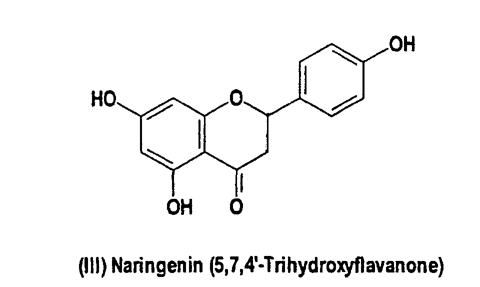 Eine bevorzugte Ausführungsform der Erfindung ist die Verwendung von Flavonen und/oder Isoflavonen, insbesondere von Genesteinen und/oder Naringeninen, die aus Adansonia- und Citrus extrahiert werden, in kosmetischen Mitteln für Haut- und Haarbehandlung.
Besonders geeignet sind Mischungen von Flavonen und Isoflavonen, wie sie vorzugsweise aus Adansonia und Citrus zugänglich sind.

Neben der Verwendung von Flavonen und Isoflavonen aus den genannten Adansoniaund Citrus-Extrakten in Kombination, ist es selbstverständlich auch möglich, die wesentlichen Bestandteile anzureichern und dann bestimmungsgemäß zu verwenden.
Die erfindungsgemäß zu verwendenden Extrakte enthalten Flavone und/oder Isoflavone in Mengen von 0,0001 - 0,01 Gew.-%, bevorzugt von 0,0001 - 0,004 Gew.-%, insbesondere von 0,0001 - 0,003 Gew.-% bezogen auf Extrakt. Bevorzugt werden Extrakte verwendet, deren Aktivsubstanzgehalt angereichert wurde in Bereiche von 0,3 - 8 Gew.-%, bevorzugt von 0,3 - 5 Gew.-%, insbesondere 0,3 - 2 Gew.-% berechnet als Aktivsubstanz, bezogen auf Extrakt.
Derartige Extrakte sind unter der Marke Vegeles® Phytofiltre AD (Laboratoires Sérobiologiques/FR) im Handel. Hierbei handelt es sich in der Regel um wäßrige oder wäßrig/alkoholische Auszüge, die man in Mengen von 0,1 bis 10, vorzugsweise 1 bis 8 und insbesondere 3 bis 5 Gew.-% - bezogen auf die Endzubereitungen - einsetzen kann, mit der Massgabe, dass sich die Mengenangaben mit Wasser und gegebenenfalls weiteren Hilfs- und Zusatzstoffen zu 100 Gew.-% addieren.

Weitere Ausführungsformen der Erfindung betreffen die Verwendungen von Flavonen und/oder Isoflavonen insbesondere von Genisteinen und Naringeninen, wobei das Gewichtsverhältnis dieser beiden Stoffgruppen untereinander 1 : (0,5 bis 5) betragen kann.
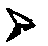 als Sonnenschutzmittel für die Haut und/oder das Haar.
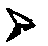 als Antioxidantien,
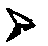 als Mittel gegen die Hautalterung.
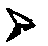 als Mittel zur Stärkung der Hautelastizität.
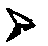 als Mittel zur Stimulation der Aktivität der Keratinocyten und Fibroblasten.

### Gewerbliche Anwendbarkeit

Die Falvone und/oder Isoflavone aus Adansonia Extrakten und Citrus-Extrakten werden in kosmetischen Mitteln für Haut- und Haarbehandlung verwendet. Diese Mittel können Emulsionen, Wachs/ Fett-Massen, Stiftpräparaten, Puder oder Salben sein. Diese Mittel können femer als weitere Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Lecithine, Phospholipide, Stabilisatoren, biogene Wirkstoffe, Deodorantien, Antitranspirantien, Antischuppenmittel, Filmbildner, Quellmittel, UV-Lichtschutz-faktoren, Antioxidantien, Hydrotrope, Konservierungsmittel, Insektenrepellentien, Selbstbräuner, Tyrosininhibitoren (Depigmentierungsmittel), Solubilisatoren, Parfümöle, Farbstoffe und dergleichen enthalten.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe, wie z.B. wie Squalan, Squalen oder Dialkylcyclohexane in Betracht.

Als **Emulgatoren** kommen beispielsweise nichtionogene Tenside aus mindestens einer der folgenden Gruppen in Frage:
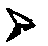 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
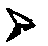 Alkyl- und/oder Alkenyloligoglykoside mit 8 bis 22 Kohlenstoffatomen im Alk(en)ylrest und deren ethoxylierte Analoga;
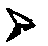 Anlagerungsprodukte von 1 bis 15 Mol Ethylenoxid an Ricinusöl und/oder gehärtetes Ricinusöl;
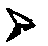 Anlagerungsprodukte von 15 bis 60 Mol Ethylenoxid an Ricinusöl und/oder gehärtetes Ricinusöl;
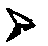 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
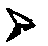 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
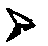 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
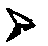 Mono-, Di- und Trialkylphosphate sowie Mono-, Di- und/oder Tri-PEG-alkylphosphate und deren Salze;
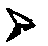 Wollwachsalkohole;
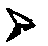 Polysiloxan-Polyalkyl-Polyether-Copolymere bzw. entsprechende Derivate;
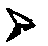 Polyalkylenglycole sowie
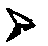 Glycerincarbonat.

Die **Anlagerungsprodukte von Ethylenoxid und/oder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

**Alkyl- und/oder Alkenyloligoglycoside,** ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Beispiele für weitere geeignete **Polyolester** sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließlich kommen auch **Kationtenside** als Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, weitere Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
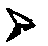 adstringierende Wirkstoffe,
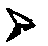 Ölkomponenten,
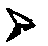 nichtionische Emulgatoren,
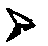 Coemulgatoren,
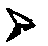 Konsistenzgeber,
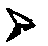 Hilfsstoffe wie z. B. Verdicker oder Komplexierungsmittel und/oder
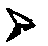 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkoniurn-Trichlorohydrat, Aluminium-Zirkonium-tetrachlorohydrat, Aluminium-Zirkonium-pentachlorohydrat und deren Komplexverbindungen z. B. mit Aminosäuren wie Glycin.
Daneben können in Antitranspirantien übliche öllösliche und wasserlösliche Hilfsmittel in geringeren Mengen enthalten sein. Solche öllöslichen Hilfsmittel können z.B. sein:
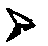 entzündungshemmende, hautschützende oder wohlriechende ätherische Öle,
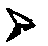 synthetische hautschützende Wirkstoffe und/oder
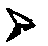 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Octopirox® (1-Hydroxy-4-methyl-6-(2,4,4-trimythylpentyl)-2-(1H)-pyridon-monoethanolaminsalz), Baypival, Pirocton Olamin, Ketoconazol®, (4-Acetyl-1-{-4-[2-(2.4-dichlorphenyl)r-2-(1H-imidazol-1-ylmethyl)-1,3-dioxylan-c-4-ylmethoxyphenyl}piperazin, Selendisulfid, Schwefel kolloidal, Schwefelpolyehtylenglykolsorbitanmonooleat, Schwefelrizinolpolyehtoxylat, Schwefelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat, Zinkpyrethion, Aluminiumpyrition und Magnesiumpyrithion / Dipyrithion-Magnesiomsulfat eingesetzt werden.

Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
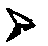 3-Benzylidencampher bzw. 3-Benzylidennorcampher und dessen Derivate, z.B. 3-(4-Methylbenzyliden)campher wie in der **EP 0693471 B1** beschrieben;
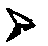 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
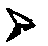 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
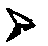 Ester der Salicylsäure, vorzugsweise Salicylsäure-2-ethylhexylester, Salicylsäure-4-isopropylbenzylester, Salicylsäurehomomenthylester,
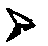 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
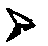 Ester der Benzalmalonsäure, vorzugsweise 4-Methoxybenzmalonsäuredi-2-ethylhexylester;
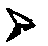 Triazinderivate, wie z.B. 2,4,6-Trianilino-(p-carbo-2'-ethyl-3'-hexyloxy)-1,3,5-triazin und Octyl Triazon, wie in der **EP 0818450 A1** beschrieben oder Dioctyl Butamido Triazone (Uvasorb® HEB);
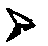 Propan-1,3-dione, wie z.B. 1-(4-tert.Butylphenyl)-3-(4'methoxyphenyl)propan-1,3-dion;
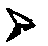 Ketotricyclo(5.2.1.0)decan-Derivaie, wie in der **EP 0694521 B**1 beschrieben.

Als wasserlösliche Substanzen kommen in Frage:
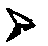 2-Phenylbenzimidazol-5-sulfonsäure und deren Alkali-, Erdalkali-, Ammonium-, Alkylammonium-, Alkanolammonium- und Glucammoniumsalze;
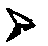 Sulfonsäurederivate von Benzophenonen, vorzugsweise 2-Hydroxy-4-methoxybenzophenon-5-sulfonsäure und ihre Salze;
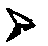 Sulfonsäurederivate des 3-Benzylidencamphers, wie z.B. 4-(2-Oxo-3-bornylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen Kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg); ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können femer **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
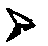 Glycerin;
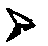 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
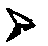 technische Oligoglyceringemische mit einem Eigenkondensationsgrad von 1,5 bis 10 wie etwa technische Diglyceringemische mit einem Diglyceringehalt von 40 bis 50 Gew.-%;
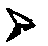 Methyolverbindungen, wie insbesondere Trimethylolethan, Trimethylolpropan, Trimethylolbutan, Pentaerythrit und Dipentaerythrit;
> Niedrigalkylglucoside, insbesondere solche mit 1 bis 8 Kohlenstoffen im Alkylrest, wie beispielsweise Methyl- und Butylglucosid;
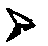 Zuckeralkohole mit 5 bis 12 Kohlenstoffatomen, wie beispielsweise Sorbit oder Mannit,
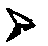 Zucker mit 5 bis 12 Kohlenstoffatomen, wie beispielsweise Glucose oder Saccharose;
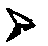 Aminozucker, wie beispielsweise Glucamin;
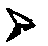 Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Ethyl Butylacetylaminopropionate in Frage, als **Selbstbräuner** eignet sich Dihydroxyaceton. Als **Tyrosinhinbitoren,** die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl, Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.
Die Sonnenschutzwirkung wird im folgenden beispielhaft dargestellt.
In den nachfolgenden Tabellen 1-4 sind eine Reihe von Formulierungsbeispielen unter Einsatz von Extrakten aus Adansonia und Citrus angegeben.

### Beispiele

### Beispiel 1: Sonnenschutzwirkung an menschlichen Haaren

Hintergrund: Für die strukturelle Einheitlichkeit des menschlichen Haares ist die chemische und physikalische Beschaffenheit von größter Wichtigkeit. Zu den Strukturmerkmalen des Haares gehört ein innerer Teil, das sogenannten Haarmark oder Medulla, welches von der Rindensubstanz, dem Cortex und der Schuppenschicht, der Kutikula umgeben ist. Die Kutikula bildet die äußere Schutzschicht und stellt damit die physikalische Barriere zu den inneren Komponenten dar. Die Kutikula spielt außerdem eine wichtige Rolle unter anderem beim Glanz des Haares, bei der Kämmbarkeit und bei Geschmeidigkeit und Volumen. Die Rindensubstanz oder Cortex liefert dem Haar mechanische Stärke wie Reissfestigkeit und Straffheit.

Umwelteinflüsse wie z.B. UV-Strahlung durch Sonneneinstrahlung verursachen schwere Schädigungen am Haar.

Methode: Eine Methode, die Schädigungen durch UV-Strahlung an menschlichem Haar zu untersuchen besteht in der Reaktion mit Dansyl-chlorid (1-Dimethylaminonaphtalene-5-sulfonyl chlorid, DANS-Cl) wie es z.B. von Sandhu und Robbins beschrieben wird (J. Soc. Cosmet. Chem., 1989, 40, 287-296).

Dansyl-chlorid ist ein typisches Sulfonyl-chlorid und reagiert mit einer großen Anzahl an Basen. Es wird genutzt als Fluoreszenz Reagenz zur Bestimmung von endständigen Aminogruppen in Peptiden und Proteinen. Das Dansyl-chlorid selbst ist nicht fluoreszierend, doch die gebildeten Derivate erscheinen mit einer intensiven gelben Fluoreszenz unter UV Licht.

Das Haar als Proteine enthaltendes Material enthält freie, N-terminale Aminogruppen und weitere Funktionalitäten, die mit Dansyl-chlorid bei unterschiedlichen pH-Werten reagieren können. Die Fluoreszenz Messung von dansyliertem Haar ergibt somit einen Nachweis von geschädigtem Haar, da sich durch die Reaktion mit Dansyl-chlorid nachweisen lässt, ob sich die Quantität der reaktiven Gruppen an den Proteinen im Haar vergrößert hat.

Die Bewertung der Schädigung an menschlichen Haaren nach UV-Strahlung wurde an standardisierten Haarsträhnen (15 cm Länge und 3 g Gewicht), welches mit dem zu testenden erfindungsgemäßen Produkt behandelt wurde, durchgeführt. Die Kontrolle entsprach unbehandeltes Haar, als Placebo diente eine wässrige Lösung eines Konservierungsmittels (0,3 % w/v). Das zu testende Produkt Vegeles Phytofiltre AD der Firma Laboratopires Sérobiologique S.A. welches Flavone und Isoflavone aus Adansonia und Citrus enthält, wurde in einer Konzentration von 4 % w/v in die Placebo Lösung eingearbeitet und getestet. Die Behandlungseffekte wurden jeweils vor und nach der Bestrahlung mit UVB-Strahlung (430 J/cm2, Strahlungsdauer 48 Stunden) und der Reaktion mit Dansyl-chlorid am Kontroll-Haar und behandeltem Haar getestet.

Test: a) Haar Reaktion mit Dansyl-chlorid: In einem Reaktionsgefäß wurden zu 50 mg Haare in 3 ml einer 0,1 M Natriumbicarbonat Lösung 1 ml frisch hergestellter Dansyl-chlorid Lösung (20 mg/ml Aceton). Die Reaktion wurde bei 37 °C im Dunkeln durchgeführt, anschließend wurden die Haarsträhnen mit Wasser und mit Aceton gewaschen um das restliche Reagenz zu entfernen. Bis zur Analyse wurden die Haarsträhnen im Dunkeln gelagert.

b) Quantitative Bestimmung der Fluoreszenz durch einen Histofluorimeter: 10 Haare jeder Haargruppe wurden parallel auf einem Objektträger angeordnet. Mit einem Histofluorimeter MPV, gekoppelt an einem optischen Mikroskop ausgestattet mit einem Reflektor für Fluoreszenz, wurde die Intensität der Fluoreszenz der zu untersuchenden Haare bestimmt. Die Intensität der Fluoreszenz wurde nach einer willkürlichen Skala von 0 (keine Fluoreszenz) bis 100 (Fluoreszenz am Kontroll-Haar ohne Behandlung) eingeteilt.

**Tabelle 1:**

| **UV-Schutzeffekt von Vegeles Phytofiltre AD an UVB-bestrahlten menschlichen Haaren durch Bestimmung der Fluoreszenz** | | |
|---|---|---|
| Probe | Ohne UVB-Strahlung | Nach UVB-Strahlung |
| Kontroll-Haar ohne Behandlung | 96,2 | 60,1 |
| Placebo Lotion | 95,2 | 67,4 |
| Lotion mit 4 % Vegeles Phytofiltre AD | 95,3 | 76,6 |

Die Fluoreszenzintentsität ist ein Maß für den Gehalt an gebundenem Dansyl-chlorid am untersuchten Haar. Jede chemische Reaktion, die die Anzahl an freien endständigen Amino Gruppen oder anderen, mit Dansyl-chlorid reagierenden Gruppen reduziert, verringert den Gehalt an gebundenem Dansyl.

Die Ergebnisse, die in der Tabelle 1 dargestellt sind, belegen die schädigende Wirkung von UVB-Strahlung am Haar durch die verringerte Fluoreszenz des Haares. Es konnte weiterhin gezeigt werden, dass die Behandlung von Haaren mit Vegeles Phytofiltre AD und damit mit Flavonen und Isoflavonen aus Adansonia und Citrus, die Verringerung der Fluoreszenz unterdrücken kann und damit die Schädigungen an menschlichen Haaren durch UVB-Strahlung reduziert.

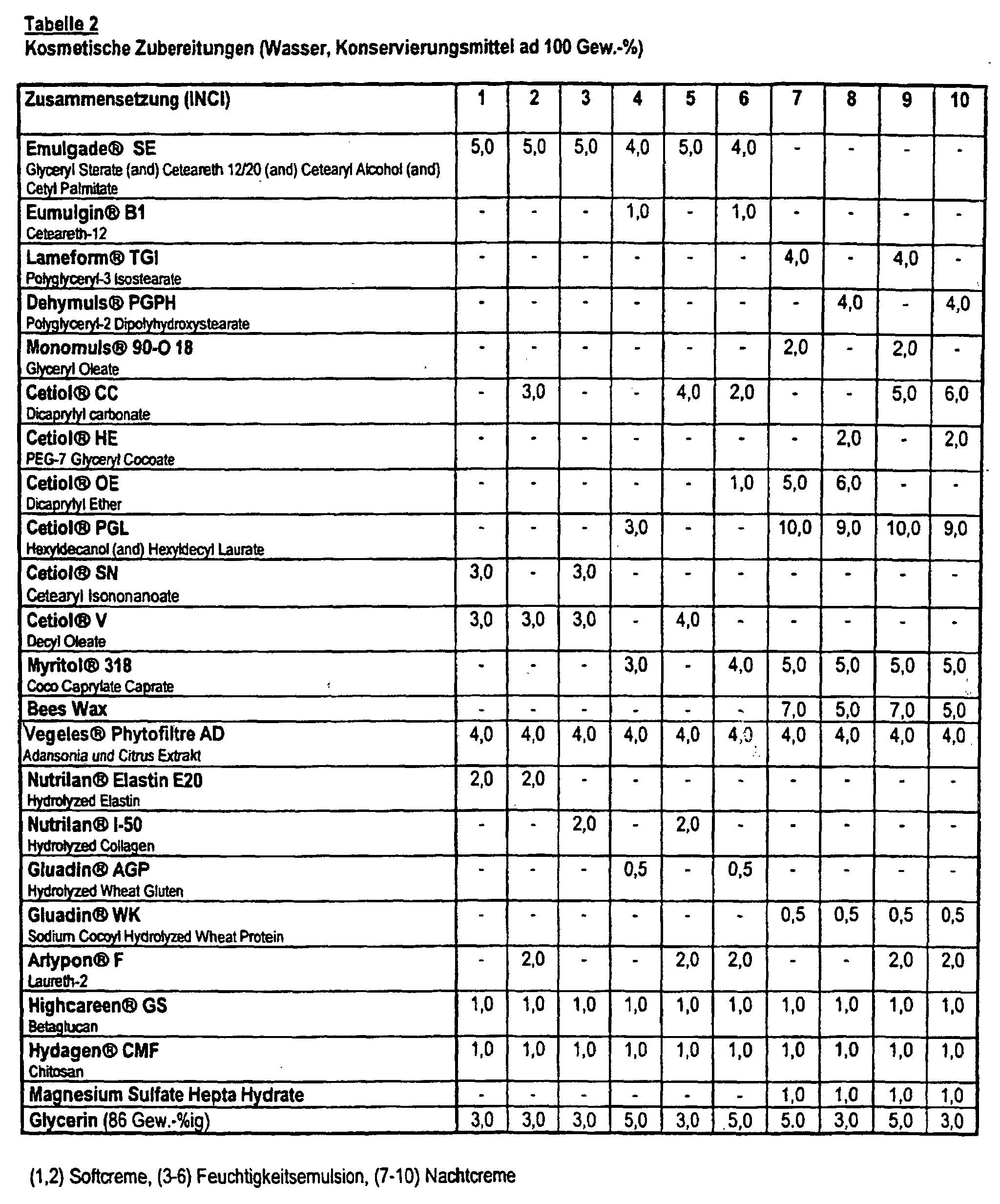

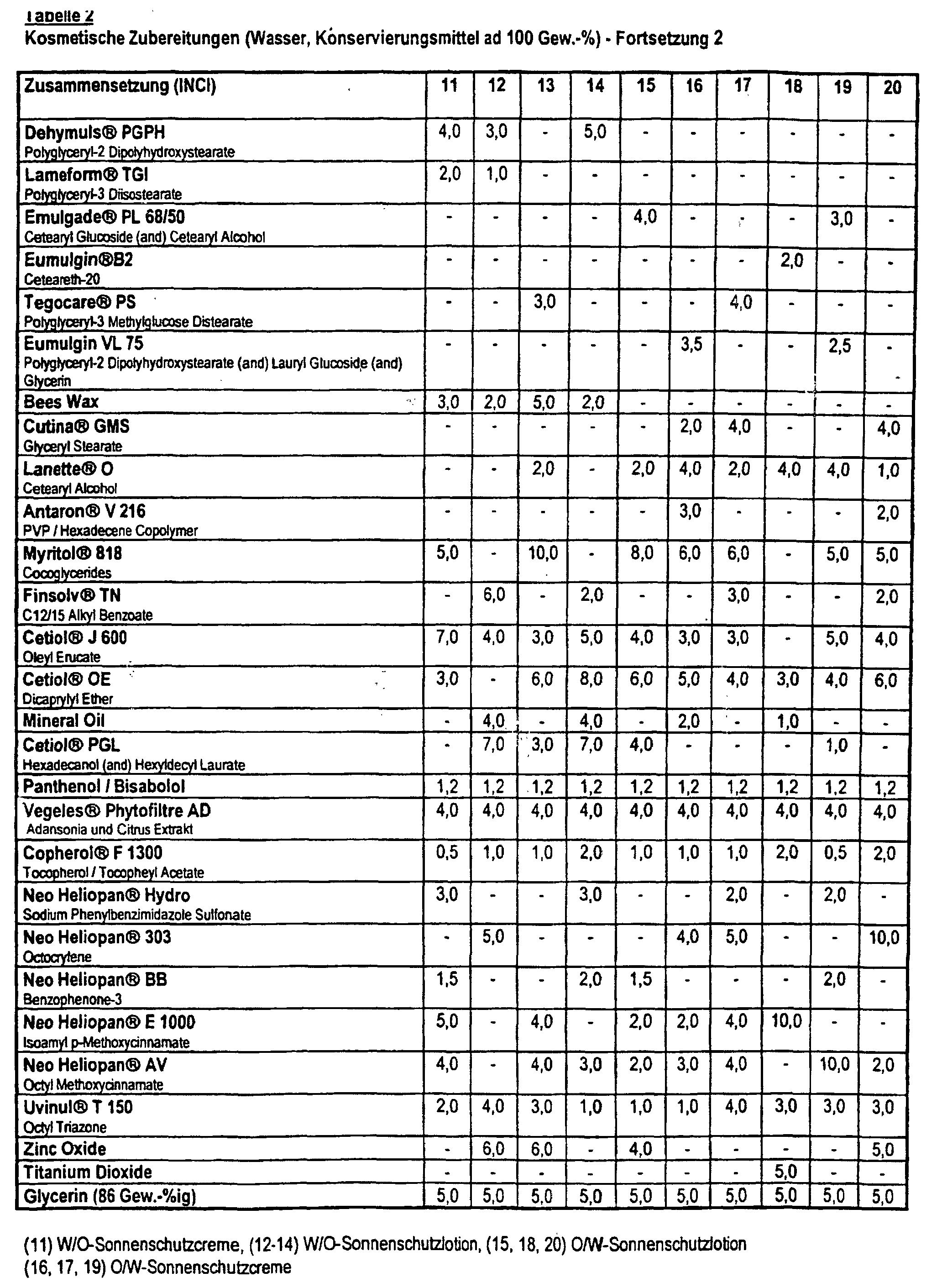

**Tabelle 3:**

| **Rezepturen für Conditioner Kosmetische Zubereitungen Conditioner (Wasser, Konservierungsmittel ad 100 Gew.-%)** | | | | | | |
|---|---|---|---|---|---|---|
| **Zusammensetzung (INCI)** | **21 Gew.%** | **22 Gew.%** | **23 Gew.%** | **24 Gew.%** | **25 Gew.%** | **26 Gew.%** |
| **Dehyquart® A** Cetrimonium Chloride | 4,0 | 4,0 | | | 3,0 | |
| **Dehyquart L® 80** Dicocoylmethylethoxymonium Methosulfate (and) Propylenglycol | | | 1,2 | 1,2 | | 1,0 |
| **Eumulgin® B2** Ceteareth-20 | 0,8 | | - | 0,8 | - | 1,0 |
| **Eumulgin® VL 75** Lauryl Glucoside (and) Polyglyceryl-2 Polyhydroxystearate (and) Glycerin | - | 2,0 | 2,0 | - | 0.8 | - |
| **Lanette® O** Cetearyl Alcohol | 3,0 | 3,0 | 3,0 | 3,0 | 3,0 | 3,0 |
| **Cutina® GMS** Glyceryl Stearate | - | 0,5 | - | 0,5 | - | 1,0 |
| **Lamesoft® PO 65** Coco-Glucosid (and) Glyceryl Oleate | | - | 3,0 | - | - | 3,0 |
| **Cetiol® J 600** Oleyl Erucate | - | 0,5 | - | 1,0 | - | 1,0 |
| **Eutanol® G** Octyldodecanol | - | - | 1,0 | - | - | 1,0 |
| **Nutrilan® Keratin W** Hydrolyzed Keratin | 5,0 | - | - | 2,0 | - | - |
| Generol® **122 N** Soja Sterol | - | - | - | - | 1,0 | 1,0 |
| **Vegeles® Phytofiltre AD** Adansonia und Citrus Extrak | 1,0 | 1,0 | 1,0 | 1,0 | 1,0 | 1,0 |
| **Copherol® 1250** Tocopheryl Acetate | - | - | 0,1 | 0,1 | - | - |
| (21-24) Haarspülung, (25-26) Haarkur | | | | | | |

**Tabelle 4:**

| **Kosmetische Zubereitungen Shampoo (Wasser, Konservierungsmittel ad 100 Gew.-%)** | | | | | | |
|---|---|---|---|---|---|---|
| **Zusammensetzung (INCI)** | **27** | **28** | **29** | **30** | **31** | **32** |
| **Texapon® NSO** Sodium Laureth Sulfate | 30,0 | | | 30,0 | 25,0 | |
| **Texapon® K 14 S** Sodium Myreth Sulfate | | 30,0 | | | | 30,0 |
| **Texapon® SB 3** Disodium Laureth Sulfosuccinate | | 10,0 | | | | |
| **Plantacare® 818** Coco Glucosides | 4,0 | | | | | |
| **Plantacare® 2000** Decyl Glucoside | | 4,0 | | | | |
| **Plantacare® PS 10** Sodium Laureth Sulfate (and) Coco Glucosides | | | 20,0 | | | |
| **Dehyton® PK 45** Cocamidopropyl Betaine | 5,0 | | | 10,0 | | 10,0 |
| **Gluadin® WK** Sodium Cocoyl Hydrolyzed Wheat Protein | | | | | 8,0 | |
| **Lamesoft® PO 65** Coco-Glucosid (and) Glyceryl Oleate | - | - | - | - | 2,0 | 2,0 |
| **Nutrilan® Keratin W** Hydrolyzed Keratin | 5,0 | - | - | - | | - |
| **Gluadin® W 40** Hydrolyzed Wheat Protein | - | 2,0 | - | 2,0 | - | - |
| **Euperlan® PK 3000 AM** Glycol Distearate (and) Laureth-4 (and) Cocamidopropyl Betaine | - | - | - | 3,0 | 3,0 | - |
| **Panthenol** | - | - | - | - | - | 0,2 |
| **Vegeles® Phylofiltre AD** Adansonia und Citrus Extrak | 1,0 | 1,0 | 1,0 | 1,0 | 1,0 | 1,0 |
| **Arlypon® F** Laureth-2 | 1,5 | - | - | - | | - - |
| **Sodium Chloride** | - | 1,6 | 2,0 | 2,2 | - | 3,0 |

## Patentansprüche

1. Verwendung von Extrakten aus Pflanzen der Gattung Adansonia und Pflanzen der Gattung Citrus, enthaltend Flavanone und/oder Isoflavone, in kosmetischen Mitteln für Haut- und/oder Haarbehandlung.

2. Verwendung von Extrakten nach Anspruch 1, **dadurch gekennzeichnet, dass** es sich bei den Flavanonen und/oder Isoflavonen um Genisteine und/oder Naringenlne handelt.

3. Verwendung von Extrakten nach Anspruch 1 und/oder 2 als Sonnenschutzmittel für die Haut und/oder das Haar.

4. Verwendung von Extrakten nach Anspruch 1 und/oder 2 als Antioxidantien.

5. Verwendung von Extrakten nach Anspruch 1 und/oder 2 als Mittel gegen die Hautalterung.

6. Verwendung von Extrakten nach Anspruch 1 und/oder 2 als Mittel zur Stärkung der Hautelastizität.

7. Verwendung von Extrakten nach Anspruch 5 und/oder 6 als Mittel zur Stimulation der Aktivität der Keratinocyten und Fibroblasten.

8. Verwendung von Extrakten nach Anspruch 1 und/oder 2, **dadurch gekennzeichnet, dass** die Extrakte auf einen Aktivsubstanzgehalt von 0,3 bis 8 Gew.% - bezogen auf den Extrakt - angereichert werden.

## Claims

1. The use of extracts of plants of the genus Adansonia and plants of the genus Citrus containing flavanones and/or isoflavones in cosmetic skin and/or hair treatment preparations.

2. The use of extracts as claimed in claim 1, **characterized in that** the flavanones and/or isoflavones are genisteins and/or naringenins.

3. The use of extracts as claimed in claim 1 and/or 2 as sun protection agents for the skin and/or hair.

4. The use of extracts as claimed in claims 1 and/or 2 as antioxidants.

5. The use of extracts as claimed in claims 1 and/or 2 as agents against ageing of the skin.

6. The use of extracts as claimed in claims 1 and/or 2 as agents for strengthening the elasticity of the skin.

7. The use of extracts as claimed in claims 5 and/or 6 as agents for stimulating the activity of keratinocytes and fibroblasts.

8. The use of extracts as claimed in claims 1 and/or 2, **characterized in that** the extracts are enriched to an active substance content of 0.3 to 8% by weight, based on the extract.

## Revendications

1. Utilisation d'extraits de plantes du genre Adansonia et de plantes du genre Citrus, contenant des flavanones et/ou des isoflavones, dans des agents cosmétiques pour le traitement de la peau et/ou des cheveux.

2. Utilisation d'extraits selon la revendication 1,
**caractérisée en ce que**
pour ce qui est des flavanones et/ou des isoflavones, il s'agit de génistéines et/ou de naringénines.

3. Utilisation d'extraits selon la revendication 1 et/ou 2, comme agents de protection solaire pour la peau et/ou les cheveux.

4. Utilisation d'extraits selon la revendication 1 et/ou 2, comme antioxydants.

5. Utilisation d'extraits selon la revendication 1 et/ou 2, comme agents contre le vieillissement de la peau.

6. Utilisation d'extraits selon la revendication 1 et/ou 2, pour le renforcement de l'élasticité de la peau.

7. Utilisation d'extraits selon la revendication 5 et/ou 6, comme agents pour la stimulation de l'activité des kératinocytes et des fibroblastes.

8. Utilisation d'extraits selon la revendication 1 et/ou 2,
**caractérisée en ce que**
les extraits sont enrichis à une teneur en substance active de 0,3 à 8 % en poids - par rapport à l'extrait.
